# EUROPÄISCHE PATENTSCHRIFT

(11) **EP 0 774 235 B1**
(45) Veröffentlichungstag und Bekanntmachung des Hinweises auf die Patenterteilung: **17.09.2003**
(21) Anmeldenummer: 96117928.0
(22) Anmeldetag: 08.11.1996
(51) Int. Cl.: A61B 6/00, A61C 19/04, G01N 21/64

(54) **Vorrichtung zum Erkennen von Karies, Plaque oder bakteriellem Befall an Zähnen**
Device for detecting caries, plaque or bacterial attack of the teeth
Dispositif destiné à la détection de la caré, de la plaque ou de l'attaque bactérielle de la denture

(30) Priorität: 08.11.1995 DE 19541686
(43) Veröffentlichungstag der Anmeldung: 21.05.1997
(73) Patentinhaber: Kaltenbach & Voigt GmbH & Co. KG, 88400 Biberach/Riss (DE)
(72) Erfinder: Hibst, Raimund, Dr., 89155 Erbach (DE); Klafke, Mario, 63814 Mainaschaff (DE); Gall, Robert, 86157 Augsburg (DE)
(74) Vertreter: Schmidt-Evers, Jürgen, Dipl.-Ing.

(56) Entgegenhaltungen:
- DE-A- 4 200 741
- DE-U- 9 317 984
- US-A- 4 479 499
- US-A- 5 369 496

## Beschreibung

Die Erfindung betrifft eine Vorrichtung zum Erkennen von Karies, Plaque oder bakteriellem Befall an Zähnen nach dem Oberbegriff des Patentanspruches 1.

Es ist bekannt, Karies, Plaque oder bakteriellen Befall an Zähnen durch visuelle Untersuchung oder durch Verwendung von Röntgenstrahlen zu entdecken. Mithilfe einer visuellen Untersuchung lassen sich jedoch häufig keine zufriedenstellenden Ergebnisse erzielen, da sich beispielsweise Karies im Frühstadium oder an einem schwer einsehbaren Zahnbereich nicht feststellen läßt. Obwohl andererseits Röntgenstrahlen sich als sehr wirksame Art zur Feststellung eines Kariesbefalls oder anderer Zahnkrankheiten herausgestellt haben, ist auch dieses Untersuchtungsverfahren aufgrund der schädigenden Wirkung der Röntgenstrahlen für die menschliche Gesundheit nicht optimal. Es bestand daher eine Notwendigkeit für die Entwicklung einer neuen Technik, um das Vorhandensein von Karies an Zähnen feststellen zu können.

In der DE 30 31 249 C2 wurde ein berührungsloses Untersuchungsverfahren zum Feststellen von Karies an menschlichen Zähnen vorgeschlagen, wobei der Zahn mit nahezu monochromatischem Licht bestrahlt wird. Die annähernd monochromatische Lichtstrahlung regt an dem Zahn eine Fluoreszenzstrahlung an. Dabei wurde entdeckt, daß das von dem Zahn reflektierte Fluoreszenzspektrum deutliche Unterschiede zwischen kariösen und gesunden Zahnbereichen aufweist. So ist im roten Spektralbereich des Fluoreszenzspektrums des Zahnes (ca. 550 bis 650 nm) die Intensität deutlich höher als bei einem gesunden Zahn. Dagegen ist im blauen Spektralbereich des reflektierten Fluoreszenzspektrums des Zahnes (ca. 350 bis 450 nm) die Intensität der Fluoreszenzstrahlung für kariöse Bereiche und gesunde Bereiche des Zahnes nahezu identisch. Die DE 30 31 249 C2 schlägt vor, den Zahn beispielsweise mit einer Wellenlänge von 410 nm zu bestrahlen und mittels zweier Filter die Fluoreszenzstrahlung des Zahnes für eine erste Wellenlänge von 450 nm sowie eine zweite Wellenlänge von 610 nm, d.h. im blauen und roten Spektralbereich, durch Fotodetektoren zu erfassen. Die durch diese Anordnung erfaßten Fluoreszenzstrahlungsintensitäten werden subtrahiert, so daß aufgrund der dadurch gewonnen Differenzintensität ein gesunder Zahnbereich eindeutig von einem kariösen Zahnbereich unterschieden werden kann.

Dasselbe Verfahren ist in S. Albin et al, "Laser Induced Fluorescence of Dental Caries", Proc SPIE 907, Seiten 96-98, 1988 beschrieben, wobei jedoch eine Anregungswellenlänge von 488 nm vorgeschlagen wird.

Die DE 42 00 741 A schlägt als vorteilhafte Weiterbildung vor, die Fluoreszenz des Zahnes durch eine Anregungsstrahlung mit einer Wellenlänge im Bereich 360 bis 580 nm hervorzurufen und die Fluoreszenzstrahlung des Zahnes für Wellenlängen ab 620 nm auszufiltern. Durch diese Maßnahmen ist der Abstand zwischen der Wellenlänge der Anregungsstrahlung und der empfangenen Fluoreszenzstrahlung ausreichend groß, so daß die Anregungsstrahlung nicht die Auswertungsergebnisse durch Überlagerung der Fluoreszenzstrahlung verfälschen kann. Mit Hilfe eines Kameran, welche die Fluoreszenzstrahlung erfaßt, wird dann ein optisches Fluoreszenzbild des Zahnes erstellt. Alternativ dazu kann die Strahlung auch mit einer Detektorvorrichtung erfaßt und das dabei erhaltene Meßsignale zur weiteren Diagnose verwertet werden.

In dem Aufsatz E. de Josselin de Jong et al, "A new Method for in vivo Quantification of Changes in Initial Enamel Caries with Laser Fluorescence", Caries Res 1995, 29, Seiten 2-7 wird vorgeschlagen, den Zahn durch Laserlicht mit einer Wellenlänge von 488 nm zu bestrahlen und die Fluoreszenzstrahlung des Zahnes für Wellenlängen ab 520 nm über eine CCD-Kamera zu erfassen und mittels eines Computerprogramms auszuwerten, um kariöse Zahnbereiche feststellen zu können.

Den zuvor beschriebenen bekannten Untersuchtungsverfahren bzw. Vorrichtungen ist gemeinsam, daß zur Anregung der Fluoreszenz eines Zahnes eine Anregungsstrahlung mit einer Wellenlänge kleiner 580 nm eingesetzt wird. Dadurch kann zwar einerseits ein verhältnismäßig hoher Wirkungsquerschnitt für die Erzeugung der Fluoreszenzstrahlung erhalten werden, doch ist die Fluoreszenzstrahlung für gesundes Zahngewebe erheblich strärker als die von kariösen Läsionen. Daher ist bei den bekannten Untersuchungsverfahren bzw. Vorrichtungen ein aufwendiger direkter Vergleich der in einem bestimmten Wellenlängenbereich vom benachbarten gesunden und kariösen Bereichen emittierten Fluoreszenzstrahlung nötig (siehe z.B. E. de Josselin de Jong et al) oder es müssen die Meßsignale der in zwei unterschiedlichen Wellenlängenbereichen erfaßten Fluoreszenzstrahlung aufwendig miteinander verglichen werden (vgl. z.B. DE 30 31 249 C2). Zudem erfordern die zuvor beschriebenen bekannten Vorrichtungen einen komplizierten Aufbau, so daß diese Geräte nicht preisgünstig hergestellt werden können und sich daher bisher kaum am Markt etabliert haben.

Allgemein nimmt mit abnehmender Wellenlänge der Anregungsstrahlung die Lichtstreuung im Zahngewebe zu. Dies führt bezüglich der bekannten Vorrichtungen und Verfahren zu einem weiteren Problem, da wegen der geringen Wellenlänge der

Anregungsstrahlung bei den bekannten Vorrichtungen und der dadurch hervorgerufenen starken Lichtstreuung nur direkt durch die Anregungsstrahlung beleuchtete Zahnoberflächenbereiche untersucht werden können.

Aus der US 4,479,499 ist daher eine Vorrichtung zum Erkennen von Karies bekannt, bei der eine Anregungsstrahlung mit einer Wellenlänge im Bereich von 600 nm verwendet wird. Wie später ausführlich erläutert wird, eignet sich eine Anregungsstrahlung in diesem Wellenlängenbereich besonders gut zur Kariesdiagnose, da unerwünschte Nebeneffekte - wird beispielsweise die Autofluoreszenz an gesundem Zahngewebe - weitestgehend entfallen, so dass eine störunanfällige Diagnose mit hoher Empfindlichkeit ermöglicht wird.

Zur Untersuchung eines bestimmten Zahnes wird dieser bei der Vorrichtung gemäß der US 4,479,499 mit zwei Anregungsstrahlungen unterschiedlicher Wellenlänge bestrahlt und die Intensität der als Antwort auf die Bestrahlung entstehenden Streu- oder Fluoreszenzstrahlung bestimmt. Die Intensitätsbestimmung erfolgt dabei für jede der beiden Anregungsstrahlungen getrennt, wobei anschließend die Differenz der beiden gemessenen Intensitäten ermittelt wird. Dieses Differenzsignal gibt Auskunft über den Gesundheitszustand des untersuchten Zahnes, da bei einem kariösen Zahngewebe eine deutlich höher Intensitätsdifferenz auftritt als bei gesundem Gewebe.

Die aus der US 4,479,499 bekannte Vorrichtung ermöglicht somit eine zuverlässige Erkennung von Karies, allerdings ist die Auswertung zweier Streu- bzw. Fluoreszenzstrahlungen, die auf unterschiedliche Anregungsstrahlungen zurückgehen, verhältnismäßig aufwendig und erfordert einen komplexen und kostenintensiven Aufbau der Diagnosevorrichtung.

Der vorliegenden Erfindung liegt deshalb die Aufgabe zugrunde, eine Vorrichtung zum Erkennen von Karies, Plaque oder bakteriellem Befall an Zähnen anzugeben, die einerseits eine hohe Empfindlichkeit aufweist, andererseits allerdings einfach, kostengünstig und störunanfällig ist.

Diese Aufgabe wird durch eine Vorrichtung gemäß den Merkmalen des Anspruchs 1 gelöst. Die erfindungsgemäße Vorrichtung zum Erkennen von Karies, Plaque oder bakteriellem Befall an Zähnen weist zunächst eine Lichtquelle zum Erzeugen einer Anregungsstrahlung auf, welche auf einen zu untersuchenden Zahn zu richten ist und an dem Zahn eine Fluoreszenzstrahlung hervorruft. Ferner ist eine Erfassungseinrichtung zum Erfassen dieser Fluoreszenzstrahlung vorgesehen, wobei die Wellenlänge der von der Lichtquelle emittierten Anregungsstrahlung zwischen 600 nm und 670 nm liegt.

Erfindungsgemäß wird aus dem von der Erfassungseinrichtung gelieferten Meßsignal unmittelbar auf das Vorhandensein bzw. Nichtvorhandensein von Karies geschlossen. Im Gegensatz zu der aus der US 4,479,499 bekannten Vorrichtung wird somit auf die aufwendige Bereitstellung zweier Anregungsstrahlungen und Ermittlung zweier Strahlungsintensitäten verzichtet und statt dessen die Intensität einer einzigen Strahlungsart zu Bewertung des Gesundheitszustands herangezogen. Die erfindungsgemäße Vorrichtung weist daher einen deutlich einfacheren Aufbau auf als die bekannte Diagnosevorrichtung, wodurch sie einerseits kostengünstiger und andererseits weniger anfällig für Störungen ist.

Vorteilhafte Ausgestaltungen der Erfindung sind in den Unteransprüchen, beschrieben.

Die erfindungsgemäße einfache Auswertung der Fluoreszenzstrahlung beruht auf experimentellen Ergebnissen, die gezeigt haben, daß sich auch mit einer Anregungsstrahlung im roten Spektralbereich (also mit einer Wellenlänge zwischen 600 nm und 670 nm) bei bakteriellem Befall von Zähnen, insbesondere bei Karies, effektiv die Fluoreszenzstrahlung des Zahnes anregen läßt. Die Anregung mit einer Strahlung in dem oben genannten Wellenlängenbereich weist den Vorteil auf, daß die Fluoreszenzstrahlung von gesunden Zahnbereichen bei derartigen Anregungswellenlängen stark abnimmt. Dadurch wird die Fluoreszenzstrahlung von kariösen Bereichen nur noch gering von der Autofluoreszenz des gesunden Zahngewebes überlagert, so daß Karies, Plaque oder bakterieller Befall an Zähnen einfach, störunanfällig und mit hoher Empfindlichkeit erkannt werden kann. Daher ist die erfindungsgemäße Vorrichtung bestens für die Frühdiagnose von Karies oder bakteriellem Befall an Zähnen geeignet.

Des weiteren kann für die Auswertung der Fluoreszenzstrahlung im Gegensatz zum bekannten Stand der Technik nicht nur ein relativ schmaler Spektralbereich der Fluoreszenstrahlung ausgenutzt werden, sondern der sehr weite Spektralbereich für Wellenlängen größer 670 nm. Zur Detektion der Fluoreszenstrahlung müssen aufgrund der hohen Empfindlichkeit der erfindungsgemäßen Vorrichtung nicht mehr aufwendige CCD-Kameras oder hochempfindliche Fotomultiplier eingesetzt werden, sondern es können zur Erfassung der Fluoreszenzstrahlung des Zahnes als lichtempfindliche Elemente einfache Fotodioden verwendet werden. Ein weiterer Vorteil der Erfindung liegt darin, daß für den erfindungsgemäß vorgeschlagenen Wellenlängenbereich des Anregungslichtes bzw. der durch das Filter erfaßten Fluoreszenzstrahlung die Streuung im Zahngewebe gering gehalten werden kann, so daß auch Karies in schwer einsehbaren oder erreichbaren Zahnbereichen, beispielsweise Karies im Zahnzwischenbereich oder unterminierende kariöse Läsionen, auf einfache Weise sicher erkannt werden kann. Schließlich ist auch vorteilhaft, daß gemäß der Erfindung einfache Lichtquellen, z.B. Laserdioden, verwendet werden können, so daß eine aufwendige Kollimatoroptik nicht mehr erforderlich ist. Ebenso ist einfacher Batteriebetrieb möglich.

Die Erfindung wird nachfolgend unter Bezugnahme auf die Zeichnung anhand bevorzugte Ausführungsbeispiele näher erläutert. Es zeigen:
Fig. 1 beispielhafte Fluoreszenzspektren für kariöses und gesundes Zahngewebe bei Anwendung der erfindungsgemäßen Vorrichtung,
Fig. 2 ein erstes Ausführungsbeispiel der erfindungsgemäßen Vorrichtung,
Fig. 3 ein zweites Ausführungsbeispiel der erfindungsgemäßen Vorrichtung,
Fig. 4 ein drittes Ausführungsbeispiel der erfindungsgemäßen Vorrichtung,
Fig. 5 eine Querschnittsansicht eines Lichtleiters der erfindungsgemäßen Vorrichtung, und
Fig. 6 eine Seitenansicht des zahnseitigen Endbereiches bei einer vorteilhaften Ausgestaltung des in Fig. 5 dargestellten Lichtleiters.

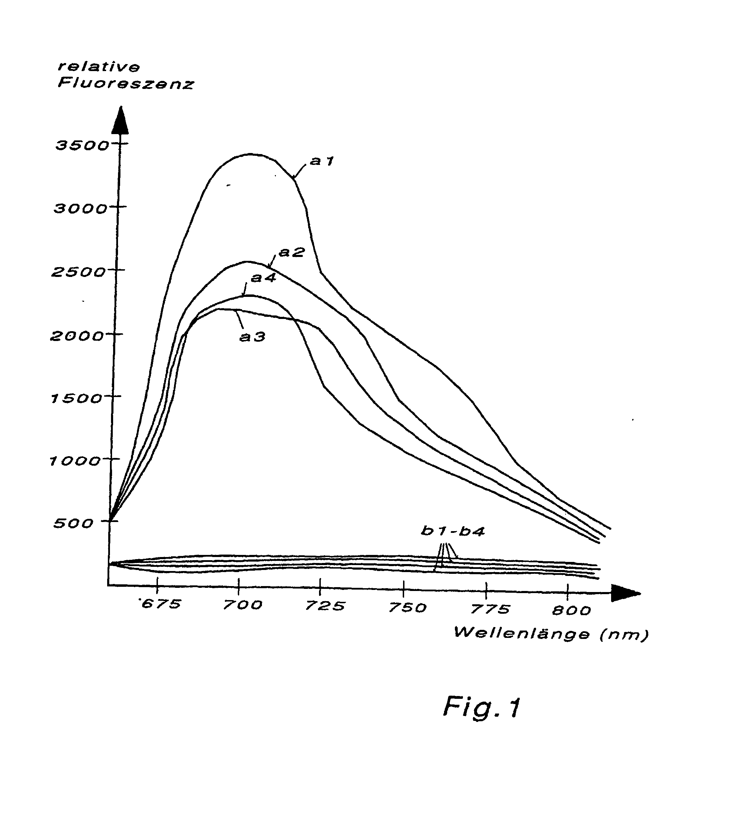

Figur 1 zeigt beispielhafte Fluoreszenspektren des Zahngewebes bei Verwendung der erfindungsgemäßen Vorrichtung. Die mit a1 bezeichneten Fluoreszenspektren stellen die Fluoreszenzspektren von kariösen Bereichen und die mit b1 bezeichneten Fluoreszenzspektren diejenigen von gesundem Zahngewebe dar. Die Fluoreszenspektren wurden durch Erzeugung einer Anregungsstrahlung mittels eines Farbstofflasers für die Anregungswellenlänge 620 nm (Fluoreszenzspektren a1 und b1), 630 nm (Fluoreszenzspektren a2 und b2), 640 nm (Fluoreszenzspektren a3 und b3) sowie 650 nm (Fluoreszenzspektren a4 und b4) erhalten. Die Laserleistung betrug für die in Figur 1 dargestellten Fluoreszenzspektren 60 mW. Aus Figur 1 ist ersichtlich, daß sich aufgrund des erfindungsgemäß vorgeschlagenen Anregungswellenlängenbereichs zwischen 600 nm und 670 nm sowie der erfindungsgemäßen Ausgestaltung der Fluoreszenzstrahlung des Zahnes für Wellenlängen größer 670 nm ein sehr großer Abstand zwischen den Fluoreszenzintensitäten für kariöse Bereiche und den Fluoreszenzintensitäten für gesunde Zahnbereiche ergibt. Das für Wellenlängen größer 670 nm erfaßte Fluoreszenzspektrum kann somit erfindungsgemäß direkt und einfach ausgewertet werden, so daß unmittelbar anhand der detektierten Fluoreszenz kariöse Bereiche nachgewiesen werden können. Die aus dem Stand der Technik bekannten sehr aufwendigen Auswertungsverfahren sind somit bei der erfindungsgemäßen Vorrichtung nicht mehr notwendig.

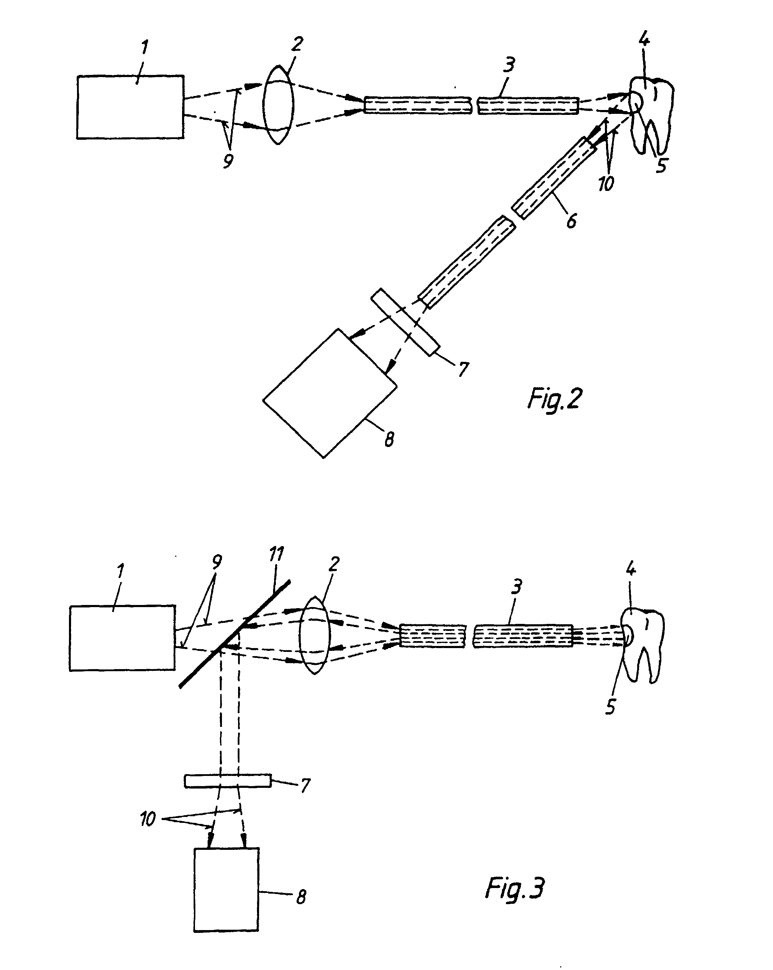

Figur 2 zeigt ein erstes Ausführungsbeispiel der erfindungsgemäßen Vorrichtung. Eine Lichtquelle 1 erzeugt eine Anregungsstrahlung 9, die über ein Einkopplungslinsensystem 2 und einen Lichtleiter einem zu untersuchenden Bereich 5 eines Zahnes 4 zugeführt wird. Durch die Anregungsstrahlung mit einer Wellenlänge im Bereich 600 nm bis 670 nm wird in dem bestrahlten Zahnbereich 5 eine Fluoreszenzstrahlung 10 über einen relativ breiten Spektralbereich hervorgerufen, die über einen zweiten Lichtleiter 6 und ein Spektralfilter 7 einer Erfassungseinrichtung 8 zum Erfassen und Auswerten der Fluoreszenzstrahlung des Zahnes zugeführt wird. Das Spektralfilter ist dabei vorzugsweise derart ausgestaltet, daß es nur für Fluoreszenzstrahlung mit einer Wellenlänge größer 670 nm durchlässig ist. Die Erfassungseinrichtung 8 wertet die ihr zugeführte Fluoreszenzstrahlung 10 direkt aus und schließt unmittelbar aus der erfaßten Fluoreszenzstrahlung 10 auf das Vorhandensein bzw. Nichtvorhandensein von Karies, Plaque oder bakteriellem Befall.

Die Lichtquelle 1 ist vorzugsweise ein HeNe-Laser oder eine Laserdiode, die die Anregungsstrahlung mit einer Wellenlänge im Bereich 600 nm bis 670 nm erzeugt. Dabei steigt mit zunehmender Wellenlänge die verfügbare Ausgangsleistung dieser Laserdioden, die Kosten nehmen ab. Dagegen verringert sich mit zunehmender Anregungswellenlänge der spektrale Unterschied zwischen der Anregungswellenlänge und der Fluoreszenstrahlung, so daß die Anforderungen an das Filter steigen. Als Kompromiß ist insbesondere eine Anregungswellenlänge von ca. 650 nm vorteilhaft.

Die Anregungsstrahlung 9 wird über ein separates Linsensystem 2 oder eine bei Laserdioden häufig schon integrierte Kollimatoroptik in den Lichtleiter 3 eingekoppelt. Ein solcher Lichtleiter kann starr oder flexibel ausgeführt sein und zudem an seinem dem Zahn zugewandten Ende mit weiteren optischen Mitteln (Linsen) zur gezielten Strahlführung ausgestattet und/oder in seinen Abmessungen an den Mundbereich des Patienten sowie an den zu untersuchenden Zahn angepaßt sein. Des weiteren können an oder in dem Lichtleiter 3 austauschbare Ablenkspiegel oder Linsen angebracht sein, die eine Untersuchung des Zahnes 4 erleichtern. Die Verwendung des Lichtleiters 3 ermöglicht es also, die Anregungsstrahlung 9 gezielt dem zu untersuchenden Bereich 5 des Zahnes bzw. der Zähne 4 zuzuführen. Dadurch kann die erfindungsgemäße Vorrichtung flexibel an verschiedene Erfordernisse in der täglichen Praxis bei der Karieserkennung an Zähnen von Menschen (oder Tieren) angepaßt werden. Das zu dem Lichtleiter 3 Gesagte gilt im gleichen Maße auch für den weiteren Lichtleiter 6, der die Fluoreszenzstrahlung dem Filter 7 zuführt. Die beiden Lichtleiter 3 und 6 können jeweils mehrere Lichtleiterfasern aufweisen. Bei Verwendung eines Lasers als Lichtquelle 1 kann die Anregungsstrahlung 9 sowie die Fluoreszenzstrahlung 10 über relativ dünne Lichtleiterfasern mit einem Kerndurchmesser von beispielsweise 200 µm übertragen werden. Die Verwendung zweier getrennter Lichtleiter 3 und 6 für die Übertragung der Anregungsstrahlung 9 bzw. der Fluoreszenstrahlung 10 ist insbesondere für die Untersuchung von Außenflächen eines Zahnes vorteilhaft. Bei dem in Figur 2 dargestellten Ausführungsbeispiel kann somit die Position der Lichtleiter 3 und 6 unabhängig voneinander und individuell am Zahn gewählt werden, was im Einzelfall eine Optimierung der Nachweisempfindlichkeit für tiefliegende oder sehr versteckt liegende Läsionen ermöglicht.

Das Filter 7 der erfindungsgemäßen Vorrichtung weist einen großen Durchlaßbereich für Wellenlängen größer 670 nm auf. Das Filter 7 kann beispielsweise durch einen Farbglas-Kantenfilter oder andere optische Elemente zur spektralen Selektion, z.B. ein Beugungsgitter, realisiert sein. Das Filter sollte vorteilhafterweise derart ausgebildet sein, daß es selber möglichst wenig fluoresziert. Wie aus Figur 1 ersichtlich ist, ist erfindungsgemäß insbesondere der Fluoreszenzspektralbereich zwischen 670 nm und 800 nm von Interesse. Daher kann ggf. in Serie mit dem Filter 7 ein weiteres Filter geschaltet sein, welches einen Sperrbereich für den langwelligen Bereich mit Wellenlängen größer 800 nm aufweist. Alternativ kann auch ein einziges Filter 7 verwendet werden, welches einen Durchlaßbereich für Wellenlängen zwischen 670 nm und 800 nm aufweist.

Die Erfassungseinrichtung 8 weist vorteilhafterweise als lichtempfindliche Elemente Fotodioden zur Detektion der Fluoreszenzstrahlung auf. Zur Erhöhung der Empfindlichkeit können die Fotodioden mit einem integrierten Vorverstärker ausgestattet sein. Ebenso kommt als Verstärkerelement in dem optischen Feld der Fluoreszenzstrahlung 10 ein Fotomuliplierer infrage.

Für den Fall, daß sowohl die Lichtquelle 1 als auch das lichtempfindliche Element der Erfassungseinrichtung 8 aus einem Halbleiterbauelement bestehen, kann zur Spannungsversorgung der erfindungsgemäßen Vorrichtung ein Niederspannungsnetzteil eingesetzt werden, welches aufgrund der geringen Leistungsaufnahme lediglich aus Batterien oder Akkus bestehen könnte.

Figur 3 zeigt ein zweites Ausführungsbeispiel der Erfindung, wobei die in Figur 2 dargestellten Bestandteile der Vorrichtung durch identische Bezugszeichen versehen sind. Bei dem in Figur 3 dargestellten Ausführungsbeispiel wird die Fluoreszenzstrahlung 10 über denselben Lichtleiter wie die Anregungsstrahlung 9 übertragen. Zum Auskoppeln der Fluoreszenzstrahlung 10 aus dem Strahlengang in dem Lichtleiter 3 ist ein Strahlteiler 11 vorgesehen, der zwischen der Lichtquelle 1 und der Linsenanordnung 2 oder alternativ zwischen der Linsenanordnung 2 und dem lichtquellenseitigen Ende des Lichtleiters 3 angeordnet ist. Diese Ausgestaltung der Erfindung ist insbesondere für den Einsatz zur Untersuchung von Wurzelkanälen von Bedeutung.

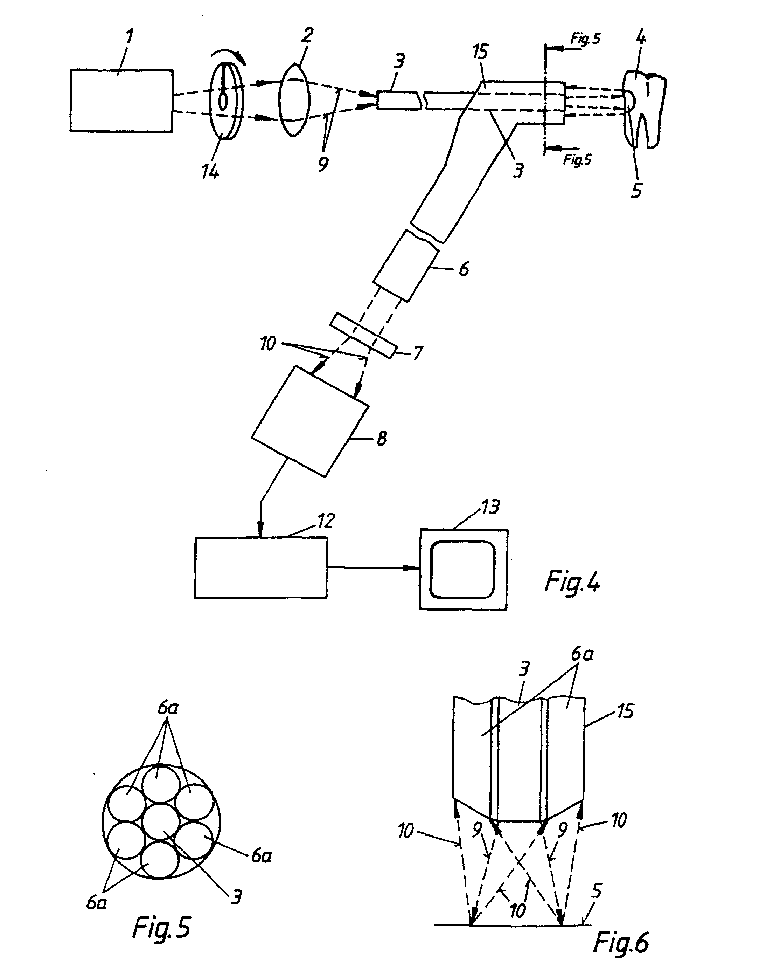

Figur 4 zeigt ein drittes Ausführungsbeispiel der erfindungsgemäßen Vorrichtung. Bei diesem Ausführungsbeispiel sind zwar im Prinzip zwei getrennte Lichtleiter 2 und 6 für die Übertragung der Anregungsstrahlung 9 bzw. der Fluoreszenzstrahlung 10 vorgesehen, jedoch ist der Lichtleiter 3 mit dem Lichtleiter 6 für eine erleichterte Handhabung zu einem Handstück 15 verbunden. Der Lichtleiter 6 selbst umfaßt mehrere Lichtleiterfasern 6a. Alternativ kann auch der Lichtleiter 3 zur Übertragung der Anregungsstrahlung 9 mehrfaserig ausgebildet sein.

Figur 5 zeigt einen Querschnitt durch das zahnseitige Ende des Handstücks 15. Vorteilhaft sind die einzelnen Lichtleiterfasern 6a des Lichtleiters 6 konzentrisch um die einzige Faser des Lichtleiters 3 angeordnet. Auf diese Weise kann die Detektionssicherheit und- genauigkeit der Fluoreszenzstrahlung erhöht bzw. stabilisiert werden.

Figur 6 zeigt eine Seitenschnittansicht des zahnseitigen Endes des Handstückes 15. Wie aus Figur 6 ersichtlich, sind vorteilhafterweise die Enden der Lichtleiterfasern 6a angeschrägt, so daß - abhängig von dem Abstand des Handstückes 15 von der zu untersuchenden Zahnoberfläche 5 - eine sichere und weitreichende Überlappung der Anregungsstrahlung 9 und der Fluoreszenzstrahlung 10 erzielt werden kann.

In Figur 4 ist zusätzlich eine Auswerteeinrichtung 12 mit einer daran gekoppelten Anzeigeeinrichtung 13 dargestellt. Die Auswerteeinrichtung 12 wertet die von der Erfassungseinrichtung 8 gelieferten Daten aus und schließt auf das Vorhandensein bzw. Nichtvorhandensein von kariösen Zahnbereichen. Die Anzeigeeinrichtung 13 dient zur visuellen Darstellung des von der Erfassungseinrichtung 8 gelieferten Meßsignales. Ebenso ist eine akustische Anzeige des Meßsignales denkbar. Die Auswerteeinrichtung 12 und/oder die Anzeigeeinrichtung 13 können in die Erfassungseinrichtung 8 aufgenommen sein.

Ein allgemein bestehendes Problem bei der Erkennung von Karies, Plaque oder bakteriellem Befall an Zähnen mit dem zuvor beschriebenen Verfahren besteht darin, daß die erfaßte Fluoreszenzstrahlung durch Tageslicht oder die künstliche Raumbeleuchtung störend überlagert werden kann. Dieses Umgebungslicht kann ebenfalls vom Zahn reflektiert und somit von den Lichtleitfasern 6a des Lichtleiters 6 erfaßt werden. Die im erfindungsgemäßen Nachweisbereich (Wellenlängen größer 670 nm) liegende spektrale Anteile des Umgebungslichts führen dann zu einem die Empfindlichkeit der Karieserkennung einschränkenden Untergrundsignal.

Dieses Problem kann erfindungsgemäß wirkungsvoll dadurch gelöst werden, daß die von der Lichtquelle 1 erzeugte Anregungsstrahlung 9 periodisch moduliert wird. So ist beispielsweise die gepulste Erzeugung der Anregungsstrahlung 9 denkbar. In diesem Fall folgt aufgrund der kurzen Lebensdauer der angeregten Zustände im Bereich von Nanosekunden die Fluoreszenstrahlung der Intensität der Anregungsstrahlung nahezu momentan. Das Umgebungslicht ist dagegen nicht periodisch moduliert und überlagert die detektierte Fluoreszenzstrahlung lediglich als Gleichanteil. Zur Auswertung der Fluoreszenzstrahlung wird nun nur die mit der entsprechenden Frequenz modulierte Strahlung als Erfassungssignal verwendet und ausgewertet. Auf diese Weise wird der Gleichanteil des Umgebungslichtes quasi ausgefiltert und die Erkennung von Karies, Plaque oder bakteriellem Befall erfolgt nahezu unabhängig von der Umgebungsbeleuchtung. Da das Umgebungslicht jedoch geringfügig mit der Frequenz der Netzspannung moduliert ist, sollte als Modulationsfrequenz für die Anregungsstrahlung 9 eine Frequenz gewählt werden, die sich von der Netzspannungsfrequenz deutlich abhebt und vorzugsweise im Bereich zwischen 100 Hz und 20 kHz liegt. Bei diesen Modulationsfrequenzen kann der modulierte Anteil der Anregungsstrahlung auf einfache Weise auch akustisch über einen Kopfhörer oder einen Lautsprecher wahrgenommen werden.

Zur Modulierung der Anregungsstrahlung 9 ist in Figur 4 eine rotierende Schlitzblende 14 dargestellt. Diese kann durch andere mechanische Chopper-Arten ersetzt werden. Wird eine Laserdiode als Lichtquelle 1 verwendet, so kann die Modulation der Anregungsstrahlung 9 auch direkt durch entsprechende Variation der Laserdiodenspannung hervorgerufen werden.

## Patentansprüche

1. Vorrichtung zum Erkennen von Karies, Plaque oder bakteriellem Befall an Zähnen,
- mit einer Lichtquelle (1) zum Erzeugen einer Anregungsstrahlung (9), welche auf einen zu untersuchenden Zahn (4) zu richten ist und an dem Zahn (4) eine Fluoreszenzstrahlung (10) hervorruft,
- mit einer Erfassungseinrichtung (8) zum Erfassen der Fluoreszenzstrahlung (10) des Zahnes (4), und
- mit einem der Erfassungseinrichtung (8) vorgeschalteten Spektralfilter (7),
wobei die Wellenlänge der von der Lichtquelle (1) emittierten Anregungsstrahlung (9) zwischen 600 nm und 670 nm liegt,
**dadurch gekennzeichnet,**
**daß** die Erfassungseinrichtung (8) die ihr zugeführte Fluoreszenzstrahlung (10) direkt auswertet und unmittelbar aus der erfaßten Fluoreszenzstrahlung (10) auf das Vorhandensein bzw. Nichtvorhandensein von Karies, Plaque oder bakteriellem Befall schließt.

2. Vorrichtung nach Anspruch 1,
**dadurch gekennzeichnet,**
**daß** das Spektralfilter (7) derart ausgestaltet ist, daß es Strahlung mit einer Wellenlänge größer 670 nm durchläßt.

3. Vorrichtung nach Anspruch 1 oder 2,
**dadurch gekennzeichnet,**
**daß** die Wellenlänge der von der Lichtquelle (1) emittierten Anregungsstrahlung (9) 630 bis 650 nm beträgt.

4. Vorrichtung nach einem der vorhergehenden Ansprüche,
**dadurch gekennzeichnet,**
**daß** das Spektralfilter (7) durch ein Farbglas-Kantenfilter oder ein Beugungsgitter realisiert ist.

5. Vorrichtung nach einem der vorhergehenden Ansprüche,
**dadurch gekennzeichnet,**
**daß** das Spektralfilter (7) schwach fluoreszierend ist.

6. Vorrichtung nach einem der vorhergehenden Ansprüche,
**dadurch gekennzeichnet,**
**daß** das Spektralfilter (7) Strahlung mit einer Wellenlänge zwischen 670 nm und 800 nm durchläßt.

7. Vorrichtung nach einem der Ansprüche 1 bis 5,
**dadurch gekennzeichnet,**
**daß** in Serie mit dem Spektralfilter (7) ein weiteres Spektralfilter angeordnet ist, welches Strahlung mit einer Wellenlänge kleiner 800 nm durchläßt.

8. Vorrichtung nach einem der vorhergehenden Ansprüche,
**dadurch gekennzeichnet,**
**daß** die Erfassungseinrichtung (8) mindestens eine Fotodiode als lichtempfindliches Element aufweist.

9. Vorrichtung nach einem der vorhergehenden Ansprüche,
**dadurch gekennzeichnet,**
**daß** die Erfassungseinrichtung (8) mit einer Auswertungseinrichtung (12) verbunden ist, welche aufgrund der durch die Erfassungseinrichtung (8) detektierten Fluoreszenzstrahlung (10) des Zahnes (4) auf das Vorhandensein bzw. Nichtvorhandensein eines kariösen Zahnes schließt.

10. Vorrichtung nach einem der vorhergehenden Ansprüche,
**dadurch gekennzeichnet,**
**daß** zur Verstärkung der Fluoreszenzstrahlung (10) des Zahnes (4) ein Verstärkerelement vorhanden ist.

11. Vorrichtung nach einem der vorhergehenden Ansprüche,
**dadurch gekennzeichnet,**
**daß** die Erfassungseinrichtung einen Fotomultiplier aufweist.

12. Vorrichtung nach einem der vorhergehenden Ansprüche,
**dadurch gekennzeichnet,**
**daß** die Anregungsstrahlung (9) und die Fluoreszenzstrahlung (10) des Zahnes (4) über Lichtleiter (3, 6) übertragen werden.

13. Vorrichtung nach Anspruch 12,
**dadurch gekennzeichnet,**
**daß** die von der Lichtquelle (1) emittierte Anregungsstrahlung (9) über ein Linsensystem (2) in den der Anregungsstrahlung (9) zugeordneten Lichtleiter (3) eingespeist und/oder über ein Linsensystem am zahnseitigen Ende des Lichtleiters (3) ausgesendet wird.

14. Vorrichtung nach Anspruch 12 oder 13,
**dadurch gekennzeichnet,**
**daß** die Fluoreszenzstrahlung (10) über ein Linsensystem in den der Fluoreszenzstrahlung (10) zugeordneten Lichtleiter (6) eingespeist und/oder über ein Linsensystem am spektralfilterseitigen Ende des Lichtleiters (6) ausgesendet wird.

15. Vorrichtung nach einem der Ansprüche 12 bis 14,
**dadurch gekennzeichnet,**
**daß** für die Anregungsstrahlung (9) und die Fluoreszenzstrahlung (10) jeweils ein separater Lichtleiter (3, 6) vorgesehen ist.

16. Vorrichtung nach einem der Ansprüche 12 bis 14,
**dadurch gekennzeichnet,**
**daß** für die Anregungsstrahlung (9) und die Fluoreszenzstrahlung (10) ein gemeinsamer Lichtleiter vorgesehen ist.

17. Vorrichtung nach Anspruche 16,
**dadurch gekennzeichnet,**
**daß** zwischen der Lichtquelle (1) und dem lichtquellenseitigen Ende des Lichtleiters (3) ein Strahlteiler (11) zum Auskoppeln der Fluoreszenzstrahlung (10) vorgesehen ist.

18. Vorrichtung nach Anspruch 16 oder 17,
**dadurch gekennzeichnet,**
**daß** der Lichtleiter mindestens eine Lichtleiterfaser (3) zum Übertragen der Anregungsstrahlung (9) sowie mehrere Lichtleiterfasern (6a) zum Übertragen der Fluoreszenzstrahlung (10) aufweist.

19. Vorrichtung nach Anspruch 18,
**dadurch gekennzeichnet,**
**daß** die Lichtleiterfasern (6a) zur Übertragung der Fluoreszenzstrahlung (10) konzentrisch um die mindestens eine Lichtleiterfaser (3) zur Übertragung der Anregungsstrahlung angeordnet sind.

20. Vorrichtung nach Anspruch 19,
**dadurch gekennzeichnet,**
**daß** die Enden der Lichtleiterfasern (6a) zum Übertragen der Fluoreszenstrahlung (10) in radialer Richtung von der die Anregungsstrahlung (9) übertragenden mindestens einen Lichtleiterfaser (3) nach außen verlaufend abgeschrägt sind.

21. Vorrichtung nach einem der Ansprüche 18 bis 20,
**dadurch gekennzeichnet,**
**daß** der Durchmesser der Lichtleiterfasern ca. 200 µm beträgt.

22. Vorrichtung nach einem der vorhergehenden Ansprüche,
**dadurch gekennzeichnet,**
**daß** die Lichtquelle (1) eine Laserdiode oder ein HeNe-Laser ist.

23. Vorrichtung nach einem der vorhergehenden Ansprüche,
**dadurch gekennzeichnet,**
**daß** die Anregungsstrahlung (9) periodisch moduliert ist, und
**daß** die Erfassungseinrichtung (8) nur eine mit der Modulationsfrequenz der Anregungsstrahlung (9) entsprechend modulierte Fluoreszenzstrahlung für den Nachweis von Karies, Plaque oder bakteriellem Befall des Zahnes (4) auswertet.

24. Vorrichtung nach Anspruch 23,
**dadurch gekennzeichnet,**
**daß** die Modulationsfrequenz der Anregungsstrahlung (9) 100 Hz bis 20 kHz beträgt.

25. Vorrichtung nach Anspruch 23 oder 24,
**dadurch gekennzeichnet,**
**daß** die periodische Modulation der Anregungsstrahlung (9) durch eine rotierende Schlitzblende (14) erfolgt.

26. Vorrichtung nach Anspruch 22 und einem der Ansprüche 23 oder 24,
**dadurch gekennzeichnet,**
**daß** die periodische Modulation der Anregungsstrahlung (9) durch Variation der an die Laserdiode angelegten Laserdiodenspannung erfolgt.

27. Vorrichtung nach einem der vorhergehenden Ansprüche,
**dadurch gekennzeichnet,**
**daß** das von der Erfassungseinrichtung (8) gelieferte Meßsignal akustisch angezeigt wird.

## Claims

1. Device for the recognition of caries, plaque or bacterial infection on teeth,
- having a light source (1) for generating an excitation radiation (9) which is to be directed onto a tooth (4) to be investigated and causes a fluorescence radiation (10) at the tooth (4),
- having a detection device (8) for detecting the fluorescence radiation (10) of the tooth (4), and
- having a spectral filter (7) before the detection device (8),
the wavelength of the excitation radiation (9) emitted from the light source (1) being between 600 nm and 670 nm,
**characterised in that**,
the detection device (8) directly evaluates the fluorescence radiation (10) delivered thereto, and determines the presence or non-presence of caries, plaque or bacterial infection immediately from the detected fluorescence radiation.

2. Device according to claim 1,
**characterised in that**,
the spectral filter (7) is so configured that it passes radiation having a wavelength greater than 670 nm.

3. Device according to claim 1 or 2,
**characterised in that**,
the wavelength of the excitation radiation (9) emitted from the light source (1) is 630 to 650 nm.

4. Device according to any preceding claim,
**characterised in that**,
the spectral filter (7) is realised by means of coloured glass cut-off filter or a diffraction grating.

5. Device according to any preceding claim,
**characterised in that**,
the spectral filter (7) is weakly fluorescent.

6. Device according to any preceding claim,
**characterised in that**,
the spectral filter (7) passes radiation having a wavelength between 670 nm and 800 nm.

7. Device according to any of claims 1 to 5,
**characterised in that**,
there is arranged in series with the spectral filter (7) a further spectral filter which passes radiation having a wavelength less than 800 nm.

8. Device according to any preceding claim,
**characterised in that**,
the detection device (8) has at least one photodiode as light sensitive element.

9. Device according to any preceding claim,
**characterised in that**,
the detection device (8) is connected with an evaluation device (12) which - on the basis of the fluorescence radiation (10) of the tooth (4) detected by means of the detection device (8) - determines the presence or non-presence of a carious tooth.

10. Device according to any preceding claim,
**characterised in that**,
an amplifier element is present for amplification of the fluorescence radiation (10) of the tooth (4).

11. Device according to any preceding claim,
**characterised in that**,
the detection device has a photomultiplier.

12. Device according to any preceding claim,
**characterised in that**,
the excitation radiation (9) and the fluorescence radiation (10) of the tooth (4) are transmitted via light conductors (3, 6).

13. Device according to claim 12,
**characterised in that**,
the excitation radiation (9) emitted from the light source (1) is fed into the light conductor (3) associated with the excitation radiation (9) via a lens system (2) and/or is delivered via a lens system at the end of the light conductor (3) towards the tooth.

14. Device according to claim 12 or 13,
**characterised in that**,
the fluorescence radiation (10) is fed into the light conductor (6) associated with the fluorescence radiation (10) via a lens system, and/or is delivered via a lens system at the end of the light conductor (6) towards to the spectral filter.

15. Device according to any of claims 12 to 14,
**characterised in that**,
respective separate light conductors (3, 6) are provided for the excitation radiation (9) and the fluorescence radiation (10).

16. Device according to any of claims 12 to 14,
**characterised in that**,
there is provided a common light conductor for the excitation radiation (9) and the fluorescence radiation (10).

17. Device according to claim 16,
**characterised in that**,
there is provided between the light source (1) and the end of the light conductor (3) towards the light source a beam divider (11) for coupling out the fluorescence radiation (10).

18. Device according to claim 16 or 17,
**characterised in that**,
the light conductor has at least one light conducting fibre (3) for transmitting the excitation radiation (9) and a plurality of light conducting fibres (6a) for transmitting the fluorescence radiation (10).

19. Device according to claim 18,
**characterised in that**,
the light conducting fibres (6a) for transmitting the fluorescence radiation (10) are arranged concentrically around the at least one light conducting fibre (3) for transmitting the excitation radiation.

20. Device according to claim 19,
**characterised in that**,
the ends of the light conducting fibres (6a) for transmitting the fluorescence radiation (10) are chamfered outwardly in radial direction from the at least one light conducting fibre (3) transmitting the excitation radiation (9).

21. Device according to any of claims 18 to 20,
**characterised in that**,
the diameter of the light conducting fibres is ca. 200 µm.

22. Device according to any preceding claim,
**characterised in that**,
the light source (1) is a laser diode or a HeNe laser.

23. Device according to any preceding claim,
**characterised in that**,
the excitation radiation (9) is periodically modulated, and **in that** the detection device (8) evaluates only a fluorescence radiation correspondingly modulated with the modulation frequency of the excitation radiation (9) for the detection of caries, plaque or bacterial infection of the tooth (4).

24. Device according to claim 23,
**characterised in that**,
the modulation frequency of the excitation radiation (9) is 100 Hz to 20 kHz.

25. Device according to claim 23 or 24,
**characterised in that**,
the periodic modulation of the excitation radiation (9) is effected by means of a rotating slit diaphragm (14).

26. Device according to claim 22 and any of claims 23 or 24,
**characterised in that**,
the periodic modulation of the excitation radiation (9) is effected by means of variation of the laser diode voltage applied to the laser diode.

27. Device according to any preceding claim,
**characterised in that**,
the measurement signal delivered from the detection device (8) is acoustically indicated.

## Revendications

1. Dispositif pour reconnaître des caries, une plaque dentaire ou l'attaque bactérienne sur les dents,
• avec une source lumineuse (1) pour produire un rayonnement d'excitation (9), laquelle doit être dirigée vers une dent (4) à inspecter et provoque un rayonnement fluorescent (10) sur la dent (4),
• avec une installation d'enregistrement (8) pour enregistrer le rayonnement fluorescent (10) de la dent (4) et
• avec un filtre spectral (7) placé en amont de l'installation d'enregistrement (8), la longueur d'onde du rayonnement d'excitation (9) émis par la source lumineuse (1) étant comprise entre 600 nm et 670 nm,
**caractérisé en ce que** l'installation d'enregistrement (8) interprète directement le rayonnement fluorescent (10) qui lui est transmis et établit immédiatement à partir du rayonnement fluorescent (10) enregistré s'il existe ou s'il n'existe pas de caries, de plaque dentaire ou d'attaque bactérienne.

2. Dispositif selon la revendication 1 **caractérisé en ce que** le filtre spectral (7) est conçu de telle manière qu'il laisse passer un rayonnement d'une longueur d'onde supérieure à 670 nm.

3. Dispositif selon la revendication 1 ou 2 **caractérisé en ce que** la longueur d'onde du rayonnement d'excitation (9) émis par la source lumineuse (1) est comprise entre 630 et 650 nm.

4. Dispositif selon l'une des revendications précédentes **caractérisé en ce que** le filtre spectral (7) est réalisé par un filtre à arêtes en verre teinté ou par un réseau de diffraction.

5. Dispositif selon l'une des revendications précédentes **caractérisé en ce que** le filtre spectral (7) est faiblement fluorescent.

6. Dispositif selon l'une des revendications précédentes **caractérisé en ce que** le filtre spectral (7) laisse passer un rayonnement d'une longueur d'onde comprise entre 670 nm et 800 nm.

7. Dispositif selon l'une des revendications 1 à 5 **caractérisé en ce qu'**un autre filtre spectral qui laisse passer un rayonnement d'une longueur d'onde inférieure à 800 nm est monté en série avec le filtre spectral (7).

8. Dispositif selon l'une des revendications précédentes **caractérisé en ce que** l'installation d'enregistrement (8) présente au moins une photodiode en tant qu'élément photosensible.

9. Dispositif selon l'une des revendications précédentes **caractérisé en ce que** l'installation d'enregistrement (8) est reliée à une installation d'interprétation (12) qui établit qu'il existe ou qu'il n'existe pas de dent cariée du fait qu'un rayonnement fluorescent (10) de la dent (4) a été détecté par l'installation d'enregistrement (8).

10. Dispositif selon l'une des revendications précédentes **caractérisé en ce qu'**il y a un élément d'intensification pour intensifier le rayonnement fluorescent (10) de la dent (4).

11. Dispositif selon l'une des revendications précédentes **caractérisé en ce que** l'installation d'interprétation présente un photomultiplicateur.

12. Dispositif selon l'une des revendications précédentes **caractérisé en ce que** le rayonnement d'excitation (9) et le rayonnement fluorescent (10) de la dent (4) sont transmis au moyen de guides d'ondes optiques (3, 6).

13. Dispositif selon la revendication 12 **caractérisé en ce que** le rayonnement d'excitation (9) émis par la source lumineuse (1) est fourni au guide d'ondes optiques (3) associé au rayonnement d'excitation (9) au moyen d'un système de lentilles (2) et/ou est envoyé à l'extrémité du guide d'ondes optiques (3) qui se trouve du côté de la dent au moyen d'un système de lentilles.

14. Dispositif selon la revendication 12 ou 13 **caractérisé en ce que** le rayonnement fluorescent (10) est fourni au guide d'ondes optiques (6) associé au rayonnement fluorescent (10) au moyen d'un système de lentilles et/ou est envoyé à l'extrémité du guide d'ondes optiques (6) qui se trouve du côté du filtre spectral au moyen d'un système de lentilles.

15. Dispositif selon l'une des revendications 12 à 14 **caractérisé en ce que** l'on prévoit respectivement un guide d'ondes optiques (3, 6) distinct pour le rayonnement d'excitation (9) et pour le rayonnement fluorescent (10).

16. Dispositif selon l'une des revendications 12 à 14 **caractérisé en ce que** l'on prévoit un guide d'ondes optiques commun pour le rayonnement d'excitation (9) et pour le rayonnement fluorescent (10).

17. Dispositif selon la revendication 16 **caractérisé en ce que** l'on prévoit entre la source lumineuse (1) et l'extrémité côté source lumineuse du guide d'ondes optiques (3) un diviseur de rayonnement (11) pour découpler le rayonnement fluorescent (10).

18. Dispositif selon la revendication 16 ou 17 **caractérisé en ce que** le guide d'ondes optiques présente au moins une fibre de guide d'ondes optiques (3) pour transmettre le rayonnement d'excitation (9) ainsi que plusieurs fibres de guide d'ondes optiques (6a) pour transmettre le rayonnement fluorescent (10)

19. Dispositif selon la revendication 18 **caractérisé en ce que** les fibres de guide d'ondes optiques (6a) pour transmettre le rayonnement fluorescent (10) sont disposées concentriquement autour de la fibre de guide d'ondes optiques (3) au moins au nombre d'une pour transmettre le rayonnement d'excitation.

20. Dispositif selon la revendication 19 **caractérisé en ce que** les extrémités des fibres de guide d'ondes optiques (6a) pour transmettre le rayonnement fluorescent (10) sont biseautées en s'étendant vers l'extérieur en direction radiale depuis la fibre de guide d'ondes optiques (3) au moins au nombre d'une qui transmet le rayonnement d'excitation (9).

21. Dispositif selon l'une des revendications 18 à 20 **caractérisé en ce que** le diamètre des fibres de guide d'ondes optiques est d'environ 200 µm.

22. Dispositif selon l'une des revendications précédentes **caractérisé en ce que** la source lumineuse (1) est une diode laser ou un laser HeNe.

23. Dispositif selon l'une des revendications précédentes **caractérisé en ce que** le rayonnement d'excitation (9) est modulé périodiquement et que l'installation d'enregistrement (8) n'interprète qu'un rayonnement fluorescent modulé en correspondance avec la modulation de fréquence du rayonnement d'excitation (9) afin de détecter des caries, une plaque dentaire ou l'attaque bactériennes de la dent (4).

24. Dispositif selon la revendication 23 **caractérisé en ce que** la modulation de fréquence du rayonnement d'excitation (9) est comprise entre 100 Hz et 20 kHz.

25. Dispositif selon la revendication 23 ou 24 **caractérisé en ce que** la modulation périodique du rayonnement d'excitation (9) est réalisée par une fente de diaphragme (14) rotative.

26. Dispositif selon la revendication 22 et l'une des revendications 23 ou 24 **caractérisé en ce que** la modulation périodique du rayonnement d'excitation (9) est réalisée en modifiant la tension de diode laser appliquée à la diode laser.

27. Dispositif selon l'une des revendications précédentes **caractérisé en ce que** le signal de mesure fourni par l'installation d'enregistrement (8) est signalé acoustiquement.
